# EUROPEAN PATENT SPECIFICATION

(11) **EP 0 371 490 B1**
(45) Date of publication and mention of the grant of the patent: **02.03.1994**
(21) Application number: 89122036.0
(22) Date of filing: 29.11.1989
(51) Int. Cl.: C12M 1/40, G01N 27/28

(54) **Measuring apparatus of two components using enzyme electrodes and the measuring method thereof**
Apparat zur Bestimmung von zwei Substanzen unter Verwendung von Enzymelektroden und Verfahren zur Ausführung dieser Bestimmung
Appareil à électrodes enzymatiques pour la mesure de deux composés et méthode pour effectuer cette mesure

(30) Priority: 30.11.1988 JP 302424/88
(43) Date of publication of application: 06.06.1990
(73) Proprietor: KANZAKI PAPER MANUFACTURING CO., LTD., Tokyo 101 (JP)
(72) Inventor: Hashizume, Yoshio, Kakogawa-shi Hyogo-ken 675 (JP); Hayashi, Ryuzo, Higashiosaka-shi Osaka 577 (JP); Kariyone, Akio, Shimogyo-ku Kyoto 600 (JP)
(74) Representative: Beetz & Partner Patentanwälte

(56) References cited:
- BIOTECHNOLOGY & BIOENGINEERING, vol. 25, 1983, John Wiley & Sons Inc., New York, NY (US); J.P. KERNEVEZ et al., pp. 845-85#
- BIOCHEMIE, no. 62, 1980; D. PFEIFFER et al., pp. 587-593#

## Description

### BACKGROUND OF THE INVENTION

### 1. Field of the Invention

The present invention relates to a measuring apparatus using enzyme electrodes for simultaneously measuring the concentration of two components such as glucose and sucrose in a liquid and its method, and more particularly to the improvement of measuring apparatus of two components and the method thereof wherein the enzymatic reaction for forming detectable substance in each electrode is common, and the substance to be finally detected by each electrode is identical.

### 2. Description of the Prior Art

Enzyme electrodes are excellent in readiness and promptness, and have come to be used widely in various fields including clinical analysis, food analysis and environmental analysis.

Recently, in particular, great efforts are concentrated in the development of measuring apparatus capable of measuring the concentration of two different substances using enzyme electrodes, and simultaneous measuring apparatus for measuring, for example, glucose and uric acid (the Japanese Laid-open Patent No. 62-24142), lactic acid and pyruvic acid (the Japanese Laid-open Patent No. 62-5172), and others have been introduced, in which each of the employed enzymes function as the catalyst for reaction with a specific object of measurement.

And also, apparatus for measuring the concentration of two components simultaneously was disclosed by the present inventors, wherein the enzymatic reaction for forming or consuming detectable substance in each electrode is common, and the substance to be finally detected by each electrode is identical (the Japanese Laid-open Patent No. 64-69944, EP0310824).

Biochemie (1980), no. 62, pages 587 to 593, D. Pfeiffer et al., discloses the measurement of two components by means of a batch method, wherein two electrodes are used, that is, an electrode including glucose oxidase and glucamylase, and an electrode including glucose oxidase. Further, it is stated therein that at first maltose is measured by bienzyme electrode, and that it is possible to measure two components of glucose and maltose at the same time.

Measuring method of sucrose is disclosed, in which the sucrose is hydrolyzed by invertase into fructose and α-D-glucose, and
α-D-glucose is converted into β-D-glucose by mutarotase, and then β-D-glucose is oxidized by glucose oxidase to produce an electrode active substance of hydrogen peroxide, which is detected electrochemically (C. Bertrand, P. R. Coulet, D. C. Gautheron, Anal. Chim. Acta, 126, 23-34, 1981). On the other hand, to measure the concentration of glucose, the glucose is oxidized by glucose oxidase, and the produced hydrogen peroxide is electrochemically detected. That is, both in the measurement of sucrose and the measurement of glucose, the oxidation of glucose is a common reaction to produce an electrode active substance, and the substance detected by the electrode is hydrogen peroxide.

Therefore, present inventors disclosed the method to measure simultaneously glucose and sucrose by using enzyme electrodes. The concentration of glucose is determined from the output voltage detected by the enzyme electrode having immobilized glucose oxidase. To measure the sucrose concentration, on the other hand, the enzyme electrode for detecting both glucose and sucrose having immobilized glucose oxidase, mutarotase and invertase is set in a flow type measuring apparatus. And by using the previously detected concentration of glucose, the contribution portion of the glucose initially contained in the sample is calculated from the preliminarily calibrated value, concerning the enzyme electrode having immobilized glucose oxidase, mutarotase and invertase, and this value is subtracted from the output current value to determine the sucrose concentration.

That is, generally, the concentrations of two components are measured in the following methods.

### Calibration means 1

First of all, using standard solutions of a first component to be measured at several concentrations, output currents are measured by a first enzyme electrode and a second enzyme electrode.

In the specification herein, the first component to be measured refers to a substance which forms by enzymatic reaction a detectable substance by the electrode. The second component to be measured, which is mentioned later, refers to a substance which does not produce an electrode detectable substance directly by the above mentioned enzymatic reaction, but is capable of producing the first component to be measured by different enzymatic reactions.

Furthermore, the first enzyme electrode is an electrode having immobilized an enzyme catalyzing the reaction to produce an electrode detectable substance from the first component to be measured. And the second enzyme electrode is an electrode having an immobilized enzyme catalyzing the reaction to produce the first component to be measured from the second component to be measured, and an immobilized enzyme catalyzing the reaction to produce an electrode detectable substance from the first component to be measured.

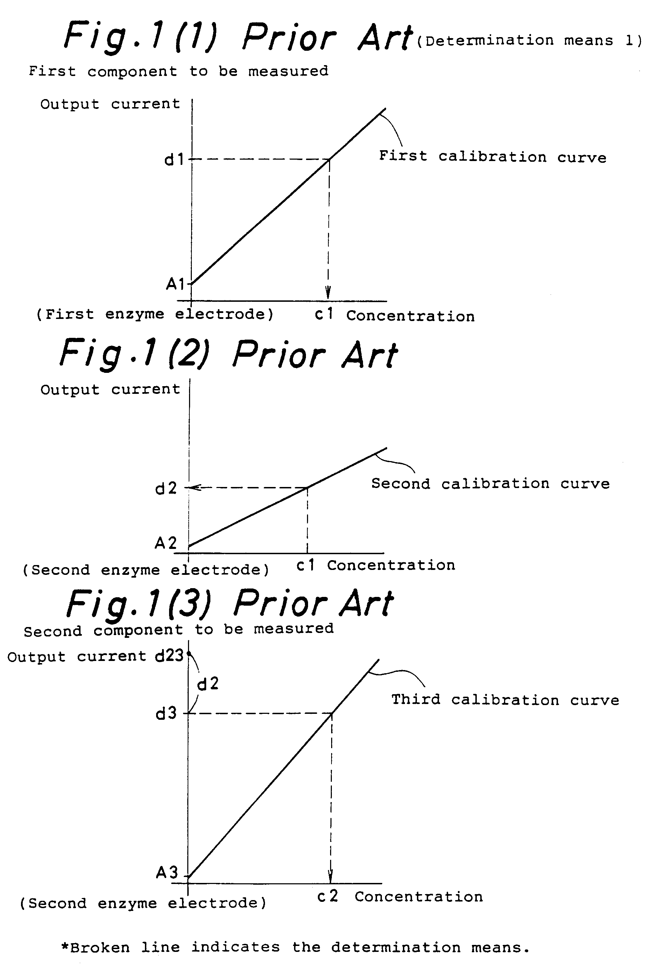

In amperometry, the concentration and output current are in proportional relation. And from the concentrations and the output current in the first enzyme electrode, a formula (1) (used as a first calibration curve for determination of the first component to be measured, as shown in Fig. 1 (1)) is obtained.

$\text{d1 = B1 x c1 + A1 (1)}$

where
- c1:: concentration of the first component to be measured
- d1:: output current of the first enzyme electrode corresponding to the first component to be measured
- A1, B1:: constants of calibration curve corresponding to the first component to be measured of the first enzyme electrode

Likewise, from the concentrations and output current in the second enzyme electrode, a formula (2) (used as a second calibration curve for detecting the output current in the second enzyme electrode of the first component to be measured, as shown in Fig. 1 (2)) is obtained.

$\text{d2 = B2 x c1 + A2 (2)}$

where
- c1:: concentration of the first component to be measured
- d2:: output current by the first component to be measured of second enzyme electrode
- A2, B2:: constants of calibration curve corresponding to the first component to be measured of the second enzyme electrode

Next, by measuring the standard solution of the second component to be measured at several concentrations by the second enzyme electrode, from the concentrations and the output current in the second enzyme electrode, a formula (3) (used as a third calibration curve for determination of the second component to be measured, as shown in Fig. 1 (3)) is obtained.

$\text{d3 = B3 x c2 + A3 (3)}$

where
- c2:: concentration of the second component to be measured
- d3:: output current by the second component to be measured of the second enzyme electrode
- A3, B3:: constants of calibration curve corresponding to the second component to be measured of the second enzyme electrode

### Determination means 1

Measuring a sample solution containing first component to be measured and second component to be measured, both of unknown concentration, by two enzyme electrodes, the output current d1 attributable to the first component to be measured is obtained in the first enzyme electrode, and the output current d23 attributable to both first component to be measured and second component to be measured is obtained in the second enzyme electrode.

The concentration c1 of the first component to be measured is obtained by putting the output current d1 in the first enzyme into formula (1), as shown in formula (a).

$\text{c1 = (d1 - A1) / B1 (a)}$

(See Fig. 1 (1).)

Next, the concentration of the second component to be measured is obtained in the following sequence. The output current of the second enzyme electrode which is attributable to the first component is calculated from the formula (2) and is obtained as correction value d2 as shown in formula (b) (see Fig. 1 (2)), and this correction value d2 is subtracted from the output current d23 obtained in the second enzyme electrode to obtain d3 (the current purely attributable to the second component to be measured) as shown in formula (c), and this value is put into formula (3) to obtained the desired concentration as shown in formula (d) (see Fig. 1 (3)).

$\text{d2 = {B2 x (d1 - A1) / B1} + A2 (b)}$

$\text{d3 = d23 - d2 (c)}$

$\text{c2 = (d3 - A3) / B3 (d)}$

The standard solution to be used in plotting of calibration curve is required to be a solution of a purely single component with a known concentration, but when measuring, for example, glucose and sucrose simultaneously as mentioned above, glucose is often contained as impurity in the standard solution of sucrose. And in the second enzyme electrode a small current derived from glucose contained in the sucrose standard solution is obtained, and therefore sucrose, which is second component to be measured, cannot be measured accurately.

Actually in a system where the substances to be finally detected by the electrodes are identical, the first component to be measured is often contained as impurity in the standard solution of the second component to be measured, and it is extremely difficult to remove this impurity or detect the content in advance, and since the substance used as the standard solution gradually produces impurities little by little as the time passes (for example, sucrose produces glucose), it is practically impossible to remove or detect the impurities preliminarily. Therefore, in the measuring method of the prior art, the correct concentration of the second component to be measured cannot be obtained. This problem becomes more serious when the sensitivity of the measuring apparatus is higher.

Presented hereabove is measurement of coexistent system of sucrose and glucose, and similar problems exist in simultaneous measuring methods of two components using enzyme electrodes producing same electrode active substances by the finally common enzymatic reactions, for example measurement of maltose-glucose coexistent system, esterified and free cholesterol coexistent system, and the like.

### SUMMARY OF THE INVENTION

It is hence a primary object of the invention to present a measuring apparatus and measuring method for determining the two components at high accuracy by solving the above-discussed problems when measuring two components common in the final enzymatic reaction for producing or consuming electrode active substances.

To achieve the above object, the two-component measuring apparatus of the invention comprises a first enzyme electrode responding only to a first component to be measured, and a second enzyme electrode responding to both the first component to be measured and the second component to be measured, both enzyme electrodes having an enzyme common in the enzymatic reaction for forming a detectable substance at electrodes, identical in the substance to be finally detected by electrodes, and further comprising means for calculating a calibration curve of the second component to be measured, by (i) calculating a contribution portion of the first component to be measured, which is an impurity in the standard solution containing a known amount of the second component, in the second enzyme electrode on the basis of a relationship (A) of the output level of the first enzyme electrode caused by a standard solution of the second component to be measured containing the first component as an impurity, and (B) the relationship of respective output levels of the first enzyme electrode and of the second enzyme electrode, caused by a standard solution of the first component to be measured; and (ii) subtracting said contribution portion from the output of the standard solution containing a known amount of the second component in the second enzyme electrode.

The invention also presents a two-component measuring apparatus which comprises:
a. a first enzyme electrode responding only to a first component to be measured;
b. a second enzyme electrode responding to both first component to be measured and second component to be measured;
c. a first memory for storing a first calibration curve for expressing the relation between the concentration of the first component to be measured and the output level of the first enzyme electrode;
d. a second memory for storing a second calibration curve for expressing the relation between the concentration of the first component to be measured and the output level of the second enzyme electrode;
e. a third memory for storing a third calibration curve for expressing the relation between the concentration of the second component to be measured and the output level of the second enzyme electrode calculated by f;
f. processing means for calculating the third calibration curve, said processing means calculates the third calibration curve on the basis of the output levels in the second enzyme electrode of the second component obtained by changing the concentration of the second component to be measured in the standard solutions, and repeats the process in the sequence of f1 to f4;
   f1. detecting the output levels attributable to the standard solution in the first enzyme electrode and the second enzyme electrode, and said standard solution contains the first component to be measured and the second component to be measured with a known concentration of the second component to be measured;
   f2. calculating the concentration of the first component to be measured on the basis of the detected output level in the first enzyme electrode and the first calibration curve stored in the first memory;
   f3. calculating the output level in the second enzyme electrode of the first component to be measured on the basis of the calculated concentration of the first component to be measured and the second calibration curve stored in the second memory;
   f4. calculating the output level in the second enzyme electrode of the second component to be measured by subtracting the calculated output level in the second enzyme electrode of the first component to be measured from the output level in the second enzyme electrode of the standard solution; and
g. processing means for calculating the concentrations of the first component to be measured and the second component to be measured of the sample by using the first, second and third calibration curves.

According to the invention, the second enzyme electrode is disposed at the downstream side of the first enzyme electrode, and
a mixing coil is disposed between the first enzyme electrode and the second enzyme electrode in order to diffuse and dilute the first and second components to be measured in the forward and backward directions of the flow.

The invention also relates to a two-component measuring method comprising:
a. a first step of preparing
   a1. a first enzyme electrode responding only to a first. component to be measured, and
   a2. a second enzyme electrode responding both to the first component to be measured and to the second component to be measured,
b. a second step of calculating a first calibration curve for expressing the relation between the concentration of the first component to be measured and the output level of the first enzyme electrode,
c. a third step of calculating a second calibration curve for expressing the relation between the concentration of the first component to be measured and the output level of the second enzyme electrode,
d. a fourth step of preparing a standard solution containing both the first component to be measured and the second component to be measured, with a known concentration of the second component to be measured,
e. a fifth step of determining the concentration of the first component to be measured contained in the standard solution by using the output level of the first enzyme electrode to the standard solution and the first calibration curve,
f. a sixth step of determining the output level in the second enzyme electrode of the first component to be measured contained in the standard solution by using the concentration of the first component to be measured and the second calibration curve,
g. a seventh step of determining the output level in the second enzyme electrode of the second component to be measured, by subtracting the output level in the second enzyme electrode of the first component to be measured from the output level of the second enzyme electrode,
h. an eighth step of calculating a third calibration curve for expressing the relation between the concentration of the second component to be measured and the output level of the second enzyme electrode, by varying the concentration of the second component to be measured in the standard solution, and repeating the procedure in the sequence of fourth step to seventh step, and
i. a ninth step of determining the concentrations of the first and second components to be measured on the basis of the first, second and third calibration curves.

In the invention, the first component to be measured delivers outputs to both first and second electrodes. Indicating the output in the first electrode of the first component to be measured with concentration c to be p1 and the output in the second electrode to be p2, the relation

$\text{c = f(p1) (A)}$

$\text{p2 = g(p1) (B)}$

is determined by measuring the standard solution of the first component to be measured, wherein f( ) and g( ) denote that c and p2 are expressed in the function of p1. If the second component to be measured is pure, no output is given to the first electrode, but if the first component to be measured is contained as impurity, a current of p1′ is delivered from this first component to be measured to the first electrode. Putting this p1′ into formula (B), the output p2′ of the second electrode due to the impurity will be calculated. Therefore, when p2′ is subtracted from the output of the second electrode of the standard solution of the second component to be measured, the second electrode output purely attributable to the second substance to be measured is obtained, so that, unlike the prior art, a very accurate calibration curve of the second component to be measured is calculated. In the conventional method, the output containing the error of p2′ attributed to the second component in the standard solution was directly taken as the output of the second component to be measured in the second electrode, and the concentration of the second component to be measured was determined by the calibration curve containing the above error and the value obtained by subtracting the output attributed to the first component to be measured, said output obtained by formula (B), from the output of the second electrode, in a measurement of sample containing first and second component to be measured. And hence the concentration was smaller than actual value. By contrast, in the invention, since the portion of the output due to the impurity is eliminated in the calculation of the calibration curve for the second component to be measured as stated above, a correct concentration of the second component to be measured is obtained.

In other words, according to the invention, in the two-component measuring apparatus comprising the first enzyme electrode responding only to the first component to be measured and the second enzyme electrode responding to both the first component to be measured and the second component to be measured, in said electrodes both contain enzyme and the enzymatic reactions for forming electrode detectable substances are identical, and the substances to be finally detected by the electrodes are identical, any determination error due to the first component to be measured which is a impurity in the second component to be measured does not occur, and therefore measurement of high sensitivity and high accuracy is realized.

### BRIEF DESCRIPTION OF THE DRAWINGS

Fig. 1 is a diagram showing a calibration curve used in a conventional two-component measuring apparatus, which is used for explaining the conventional means for determining;
Fig. 2 is a system diagram showing an example of a two-component measuring apparatus of the invention;
Fig. 3 is a diagram showing a calibration curve used in a two-component measuring apparatus of the invention, which is used for explaining the method of plotting a third calibration curve; and

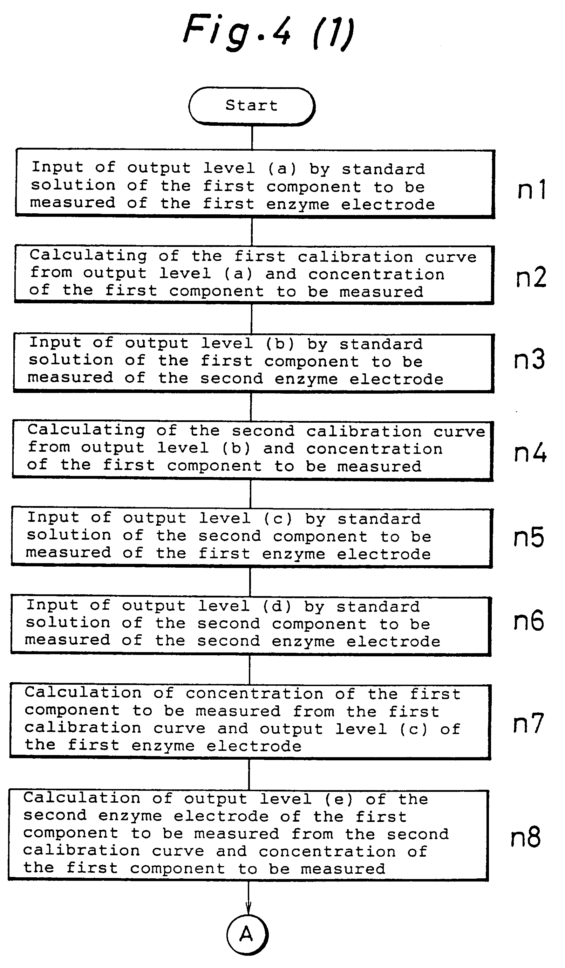

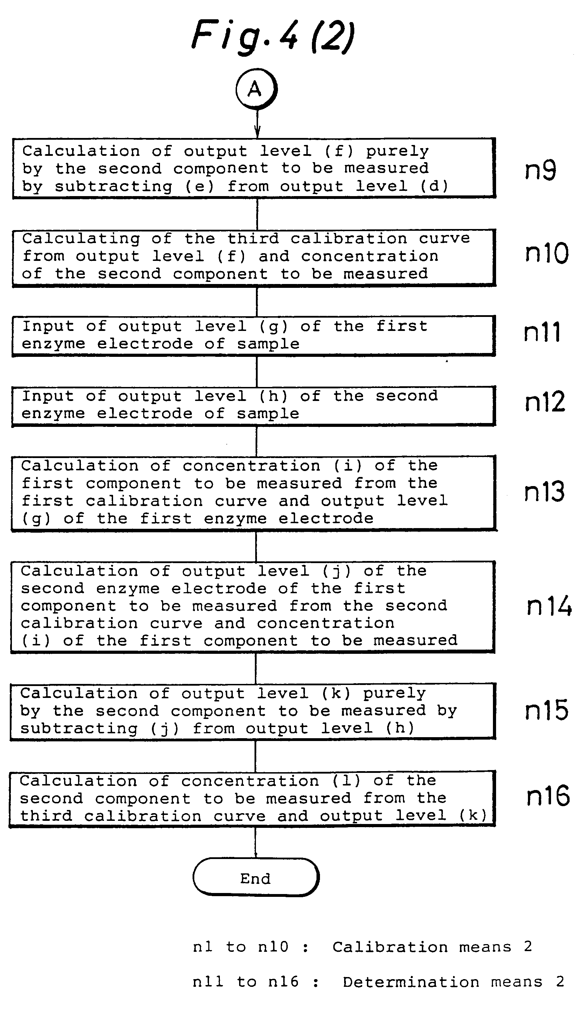

Fig. 4 is a flow chart showing an arithmetic processing unit of the system diagram shown in Fig. 2.

### DETAILED DESCRIPTION OF THE PREFERRED EMBODIMENTS

Preferred embodiments of the invention are described in details with reference to the drawings attached hereto.

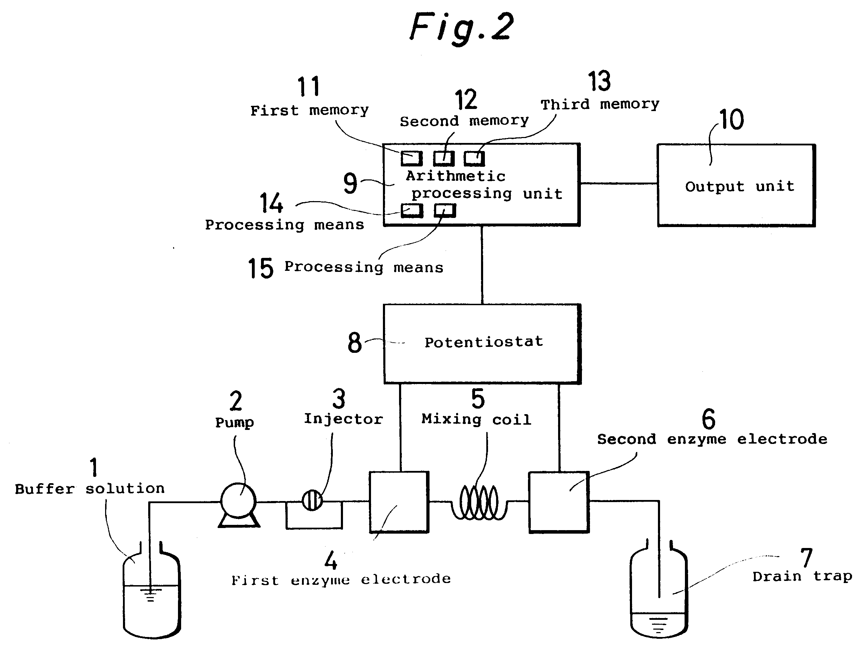

Fig. 2 is a system diagram showing an example of a two-component measuring apparatus of the invention. This embodiment shows a flow type, in which a buffer solution 1 is supplied through a pump 2, the substance to be measured is injected from an injector 3, and it is sent out into a drain trap 7 from a first enzyme electrode 4 through a mixing coil 5 and a second enzyme electrode 6.

To each electrode, a voltage is applied by a potentiostat 8, and a current output based on each component to be measured is obtained, and this information is sent to an arithmetic processing unit 9 by means for generally converting trace current into a digital quantity such as current amplification and by digitizing the output current with an A/D converter, and the operation for determination of calibration curves and concentration is executed, and the result is delivered in a displaying or printing output unit 10. In order to explain, the first memory, the second memory and the third memory were described separately, but they can be memorized at specific address of the same Random Access memory.

By diluting the sample, for example, by intervening a mixing coil 5 between the first enzyme electrode and the second enzyme electrode, the proportional range of the second enzyme electrode is expanded, and the two substances may be measured more accurately even if the concentration is very high (refer to the Japanese Laid-open Patent No. 64-69944, EP0310824).

As detecting methods in measurement, in addition to the amperometric method above mentioned, potentiometric method and thermal measurement method (to measure the changes in enthalpy due to enzymatic reaction) may be possible, and considering from the dynamic range and response speed of these methods, the amperometric method is most preferable.

In the invention, the calibration means in the arithmetic processing unit 9 may be calibrated in the procedure shown in, for example, Calibration means 2 below, and thereafter the sample of unknown concentration is measured in the procedure shown in Determination means 2 below.

### Calibration means 2

First of all, output levels of a standard solution of the first component to be measured at several concentrations are measured by the first enzyme electrode and second enzyme electrode.

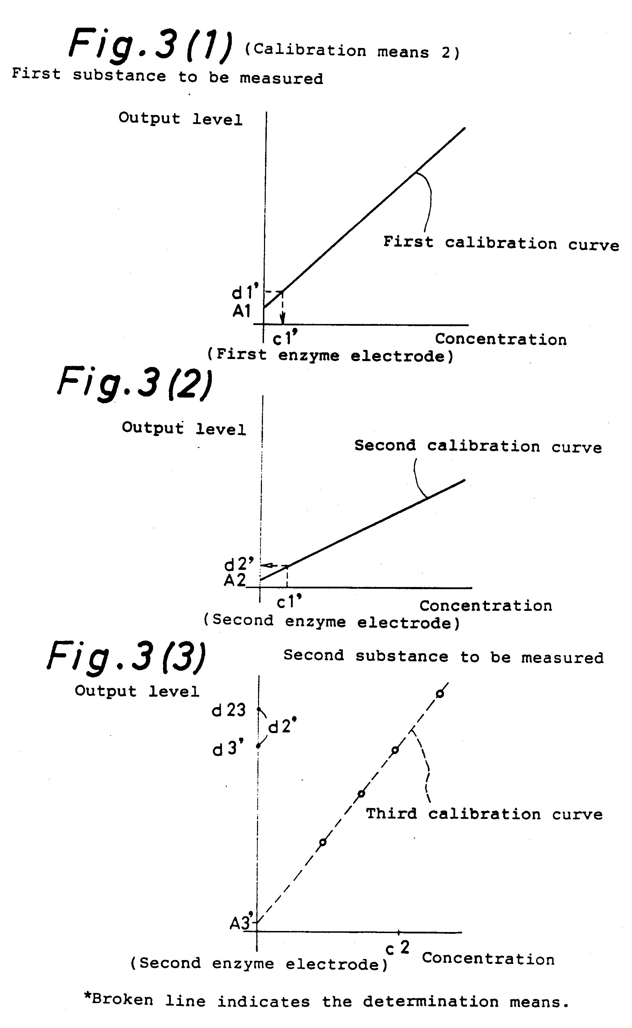

From the concentrations and the output levels in the first enzyme electrode, formula (4) (used as a first calibration curve for determination of the first component to be measured, as shown in Fig. 3 (1)) is obtained.

$\text{d1 = B1 x c1 + A1 (4)}$

where
- c1:: concentration of the first component to be measured
- d1:: output level of the first enzyme electrode to the first component to be measured
- A1, B1:: constants of calibration curve of the first enzyme electrode to the first component to be measured

Likewise, from the concentrations and output levels in the second enzyme electrode, formula (5) (used as a second calibration curve for detecting the output level in the second enzyme electrode of the first component to be measured, as shown in Fig. 3 (2)) is obtained.

$\text{d2 = B2 x c1 + A2 (5)}$

where
- c1:: concentration of the first component to be measured
- d2:: output level of the second enzyme electrode attributed to the first component to be measured
- A2, B2:: constants of calibration curve of the second enzyme electrode attributed to the first component to be measured

Next, using a standard solution of the second component to be measured at known concentration (c2), the output level d1 attributable to the first component to be measured contained as impurity is measured in the first enzyme electrode, and the output level d23 attributable to both the second component to be measured and the first component to be measured contained as impurity is measured in the second enzyme electrode.

The contributing portion d2′ of the first component to be measured in the second enzyme electrode is obtained from formula (4) and (5) (see Fig. 3 (1), (2)),

$\text{d2′ = {B2 x (d1′ - A1) / B1} + A2}$

and the contributing portion d2′ is subtracted from the output current d23 in the second enzyme electrode, thereby calculating d3′ as follows:

$\text{d3′ = d23 - d2′}$

Similarly measuring standard solutions of the second component to be measured at several concentrations, formula (6) about the corrected output current and concentration (used as a third calibration curve for determination of second component to be measured, as shown in Fig. 3 (3)) is obtained.

$\text{d3′ = B3′ x c2 + A3′ (6)}$

where
- c2:: concentration of the second component to be measured
- d3′:: true output level of the second enzyme electrode attributable to the second component to be measured
- A3′, B3′:: constants of calibration curve of the second enzyme electrode to the second component to be measured

Thus, the first, second and third calibration curves are plotted automatically, and are stored in first, second and third memories in the arithmetic processing unit. The calibration curves may be used in a certain small error range as far as the experimental conditions are not changed.

However, the enzymatic reaction changes its rate when the pH of the buffer solution is changed only slightly even if the temperature, sample volume and flow rate are constant. In actual measurement, considering conditions of the parts of the apparatus, for example, temperature fluctuations in the detecting unit due to ambient temperature or slight variation of the pump flow rate, it is desired to calculate the calibration curve just before measurement of the sample from the viewpoint of measuring at high precision.

Incidentally, although different due to the capacity of the memory and purpose of use of the apparatus, it may be also possible to store the calibration curves for plural sets of objects of measurement, and select and use proper calibration curves when the electrodes are exchanged. In this method, however, due caution is needed because the number of condition fluctuation factors may increase.

### Determination means 2

A sample solution containing the first component to be measured and the second component to be measured, both of unknown concentrations, is measured by two enzyme electrodes, and the output level d1 attributable to the first component to be measured is obtained in the first enzyme electrode, and the output level d23 attributable to both the first component to be measured and the second component to be measured is obtained in the second enzyme electrode.

The concentration of the first substance to be measured c1 is obtained by putting the output level d1 in the first enzyme electrode into formula (4) as shown in formula (a).

$\text{c1 = (d1 - A1) / B1 (a)}$

Next, the concentration of the second component to be measured is obtained by first calculating the output level expected for the first component to be measured in the second enzyme electrode from the formula (5) as shown in formula (b) to be obtained as correction value d2. Then subtracting this correction value d2 from the output level d23 obtained in the second enzyme electrode as shown in formula (c) to obtain d3 (the level purely attributable to the second substance to be measured), and putting this value in formula (6) as shown in formula (d).

$\text{d2 = {B2 x (d1 - A1) / B1} + A2 (b)}$

$\text{d3 = d23 - d2 (c)}$

$\text{c2 = (d3 - A3) / B3 (d)}$

The invention of two-component measuring apparatus was conducted, by the amperometric measurement according to the Calibration means 2, using distilled water as control sample and also using glucose standard solutions of 10, 20, 300 mmol/ℓ, and sucrose standard solutions of 10, 20, 30 mmol/ℓ. And said apparatus was installed with the first enzyme electrode having immobilized glucose oxidase, and the second enzyme electrode having immobilized invertase, mutarotase and glucose oxidase. Afterwards, instead of using the solutions of unknown concentrations, using again the glucose standard solutions of 10, 20, 30 mmol/ℓ and sucrose standard solutions of 10, 20, 30 mmol/ℓ, each measurement was repeated three times according to the Determination means 2, and the results are shown in Table 1.

Besides, by way of comparison, the results of measurement according to the Calibration means 1 and Determination means 1 which lacks correction of the calibration curve of the second component to be measured are shown in Table 2.

As known from Table 1 and 2, in the measurement employing the two-component measuring apparatus of the invention, the error of the determination of the second substance to be measured has been notably improved.

**Table 1**

| Substance measured | Results of measurement (Unit: mmol/ℓ) | | | | | |
|---|---|---|---|---|---|---|
| | Glucose | | | Sucrose | | |
| | 1st | 2nd | 3rd | 1st | 2nd | 3rd |
| 10 mmol/ℓ glucose | 10.12 | 9.85 | 9.8 | 0.02 | 0.35 | 0.40 |
| 20 mmol/ℓ glucose | 20.15 | 19.97 | 19.89 | 0.00 | 0.11 | 0.37 |
| 30 mmol/ℓ glucose | 30.16 | 29.94 | 29.73 | 0.00 | 0.00 | 0.00 |
| 10 mmol/ℓ sucrose | 0.39 | 0.37 | 0.33 | 10.18 | 9.99 | 9.88 |
| 20 mmol/ℓ sucrose | 0.78 | 0.79 | 0.75 | 20.08 | 19.91 | 19.59 |
| 30 mmol/ℓ sucrose | 0.65 | 0.68 | 0.68 | 29.75 | 30.34 | 30.00 |

**Table 2**

| Substance measured | Results of measurement (Unit: mmol/ℓ) | | | | | |
|---|---|---|---|---|---|---|
| | Glucose | | | Sucrose | | |
| | 1st | 2nd | 3rd | 1st | 2nd | 3rd |
| 10 mmol/ℓ glucose | 10.12 | 9.85 | 9.81 | 0.00 | 0.15 | 0.19 |
| 20 mmol/ℓ glucose | 20.15 | 19.97 | 19.89 | 0.00 | 0.00 | 0.16 |
| 30 mmol/ℓ glucose | 30.16 | 29.94 | 29.73 | 0.00 | 0.00 | 0.00 |
| 10 mmol/ℓ sucrose | 0.39 | 0.37 | 0.33 | 9.65 | 9.46 | 9.36 |
| 20 mmol/ℓ sucrose | 0.78 | 0.79 | 0.75 | 19.21 | 19.05 | 20.01 |
| 30 mmol/ℓ sucrose | 0.65 | 0.68 | 0.68 | 29.46 | 29.14 | 28.81 |

The invention has been hitherto mainly illustrated in the measurement of sucrose and glucose coexistent system, but the invention also brings about similar effects by using appropriate enzyme electrodes even in simultaneous measurement of two components using enzyme electrodes producing same electrode active substance by the finally common enzymatic reactions in maltose and glucose coexistent system, esterified and free cholesterol coexistent system, and others.

That is, if the first component is glucose and the second component is sucrose, the first enzyme electrode is an electrode having immobilized glucose oxidase, and the second enzyme electrode is an electrode having immobilized glucose oxidase, mutarotase and invertase, as stated above.

Besides, if the first component is glucose and the second component is maltose, the first enzyme electrode is an electrode having immobilized glucose oxidase, and the second enzyme electrode is an electrode having immobilized glucose oxidase, mutarotase and α-glucoxidase.

If the first component is glucose and the second component is β-glucoside, the first enzyme electrode is an electrode having immobilized glucose oxidase, and the second enzyme electrode is an electrode having immobilized glucose oxidase and β-glucosidase.

If the first component is glucose and the second component is maltooligosugars, the first enzyme electrode is an electrode having immobilized glucose oxidase, and the second enzyme electrode is an electrode having immobilized glucose oxidase and glucoamylase.

If the first component is glucose and the second component is lactose, the first enzyme electrode is an electrode having immobilized glucose oxidase, and the second enzyme electrode is an electrode having immobilized glucose oxidase and lactase.

For detection of free cholesterol and esterified cholesterol, the first enzyme electrode is an electrode having immobilized cholesterol oxidase, and the second enzyme electrode is an electrode having immobilized cholesterol oxidase and cholesterol esterase.

## Claims

1. A two-component measuring apparatus comprising: a first enzyme electrode (4) responding only to a first component to be measured, and a second enzyme electrode (6) responding to both the first component to be measured and the second component to be measured, both enzyme electrodes (4, 6) having an enzyme common in the enzymatic reaction for forming a detectable substance at the electrodes, identical in the substance to be finally detected by the electrodes, and further comprising means for calculating a calibration curve of the second component to be measured, by:
(i) calculating a contribution portion of the first component to be measured, which is an impurity in the standard solution containing a known amount of the second component, in the second enzyme electrode (6) on the basis of a relationship of
(A) the output level of the first enzyme electrode (4) caused by a standard solution of the second component to be measured containing the first component as an impurity, and
(B) the relationship of respective output levels of the first enzyme electrode (4) and of the second enzyme electrode (6), caused by a standard solution of the first component to be measured; and
(ii) subtracting said contribution portion from the output of the standard solution containing a known amount of the second component in the second enzyme electrode (6).

2. A two-component measuring apparatus comprising:
a. a first enzyme electrode (4) responding only to a first component to be measured;
b. a second enzyme electrode (6) responding to both first component to be measured and second component to be measured;
c. a first memory (11) for storing a first calibration curve for expressing the relation between the concentration of the first component to be measured and the output level of the first enzyme electrode (4);
d. a second memory (12) for storing a second calibration curve for expressing the relation between the concentration of the first component to be measured and the output level of the second enzyme electrode (6);
e. a third memory (13) for storing a third calibration curve for expressing the relation between the concentration of the second component to be measured and the output level of the second enzyme electrode (6) calculated by f;
f. processing means (14) for calculating the third calibration curve, said processing means (14) calculating the third calibration curve on the basis of the output levels in the second enzyme electrode (6) of the second component obtained by changing the concentration of the second component to be measured in a standard solution, and repeating the process in the sequence of f1 to f4;
f1. detecting the output levels attributable to the standard solution in the first enzyme electrode (4) and the second enzyme electrode (6), said standard solution containing the first component to be measured and the second component to be measured with a known concentration of the second component to be measured;
f2. calculating the concentration of the first component to be measured on the basis of the detected output level in the first enzyme electrode (4) and the first calibration curve stored in the first memory (11);
f3. calculating the output level in the second enzyme electrode (6) of the first component to be measured on the basis of the calculated concentration of the first component to be measured and the second calibration curve stored in the second memory (12);
f4. calculating the output level in the second enzyme electrode (6) of the second component to be measured by subtracting the calculated output level in the second enzyme electrode (6) of the first component to be measured from the output level in the second enzyme electrode (6) of the standard solution; and
g. processing means (15) for calculating the concentrations of the first component to be measured and the second component to be measured of the sample by using the first, second and third calibration curves.

3. A two-component measuring apparatus according to claim 1 or 2, wherein the second enzyme electrode (6) is disposed at the downstream side of the first enzyme electrode (4), and
a mixing coil (5) is disposed between the first enzyme electrode (4) and the second enzyme electrode (6) in order to diffuse and dilute the first and second components to be measured in the forward and backward directions of the flow.

4. A two-component measuring method comprising:
a. a first step of preparing
a1. a first enzyme electrode (4) responding only to a first component to be measured, and
a2. a second enzyme electrode (6) responding both to the first component to be measured and to the second component to be measured,
b. a second step of calculating a first calibration curve for expressing the relation between the concentration of the first component to be measured and the output level of the first enzyme electrode (4),
c. a third step of calculating a second calibration curve for expressing the relation between the concentration of the first component to be measured and the output level of the second enzyme electrode (6),
d. a fourth step of preparing a standard solution containing both the first component to be measured and the second component to be measured, with a known concentration of the second component to be measured,
e. a fifth step of determining the concentration of the first component to be measured contained in the standard solution by using the output level of the first enzyme electrode (4) to the standard solution and the first calibration curve,
f. a sixth step of determining the output level in the second enzyme electrode (6) of the first component to be measured contained in the standard solution by using the concentration of the first component to be measured and the second calibration curve,
g. a seventh step of determining the output level in the second enzyme electrode (6) of the second component to be measured, by subtracting the output level in the second enzyme electrode (6) of the first component to be measured from the output level of the second enzyme electrode (6),
h. an eighth step of calculating a third calibration curve for expressing the relation between the concentration of the second component to be measured and the output level of the second enzyme electrode (6), by varying the concentration of the second component to be measured in the standard solution, and repeating the procedure in the sequence of fourth step to seventh step, and
i. a ninth step of determining the concentrations of the first and second components to be measured on the basis of the first, second and third calibration curves.

## Patentansprüche

1. Zweikomponentenmeßvorrichtung umfassend eine erste Enzymelektrode (4), die nur auf eine zu messende erste Komponente anspricht, und eine zweite Enzymelektrode (6), die sowohl auf die zu messende erste Komponente als auch auf die zu messende zweite Komponente anspricht, wobei beide Enzymelektroden (4, 6) ein Enzym aufweisen und die enzymatischen Reaktionen zur Bildung einer an den Elektroden ermittelbaren Substanz sowie die durch die Elektroden schließlich ermittelten Substanzen identisch sind, und ferner umfassend eine Einrichtung zum Berechnen einer Eichkurve der zu messenden zweiten Komponente durch
(i) Berechnen eines Beitrags der zu messenden ersten Komponente, die eine Verunreinigung in der eine bekannte Menge der zweiten Komponente enthaltenden Standardlösung ist, in der zweiten Enzymelektrode (6) auf der Basis einer Beziehung
(A) des Ausgangsniveaus der ersten Enzymelektrode verursacht durch eine Standardlösung der zu messenden zweiten Komponente, die die erste Komponente als Verunreinigung enthält, und
(B) der Beziehung der jeweiligen Ausgangsniveaus der ersten Enzymelektrode und der zweiten Enzymelektrode (6), verursacht durch eine Standardlösung der zu messenden ersten Komponente, und
(ii) Subtrahieren des Beitrags vom Ausgang der Standardlösung, die eine bekannte Menge der zweiten Komponente in der zweiten Enzymelektrode enthält.

2. Zweikomponentenmeßvorrichtung umfassend:
(a) eine erste Enzymelektrode, die nur auf eine zu messende erste Komponente anspricht,
(b) eine zweite Enzymelektrode (6), die sowohl auf die zu messende erste Komponente als auch auf die zu mes sende zweite Komponente anspricht,
(c) einen ersten Speicher (11) zum Speichern einer ersten Eichkurve für die Beziehung zwischen der Konzentration der zu messenden ersten Komponente und dem Ausgangsniveau der ersten Enzymelektrode (4),
(d) einen zweiten Speicher (12) zum Speichern einer zweiten Eichkurve für die Beziehung zwischen der Konzentration der zu messenden ersten Komponente und dem Ausgangsniveau der zweiten Enzymelektrode (6),
(e) einen dritten Speicher (13) zum Speichern einer dritten Eichkurve für die Beziehung zwischen der Konzentration der zu messenden zweiten Komponente und dem durch (f) berechnenten Ausgangsniveau der zweiten Enzymelektrode (6),
(f) eine Verarbeitungseinrichtung (14) zum Berechnen der dritten Eichkurve, wobei die Verarbeitungseinrichtung (14) eine dritte Eichkurve berechnet auf der Basis der Ausgangsniveaus in der zweiten Enzymelektrode (6) der zweiten Komponente, erhalten durch Ändern der Konzentration der zu messenden zweiten Komponente in einer Standardlösung und durch Wiederholen des Prozesses aufeinanderfolgend von (f1) bis (f4),
(f1) Ermitteln der Ausgangsniveaus, die der Standardlösung in der ersten Enzymelektrode (4) und der zweiten Enzymelektrode (6) zugeschrieben werden können, wobei die Standardlösung die zu messende erste Komponente und die zu messende zweite Komponente enthält mit einer bekannten Konzentration der zu messenden zweiten Komponente,
(f2) Berechnen der Konzentration der zu messenden ersten Komponente auf der Basis des ermittelten Ausgangsniveaus in der ersten Enzymelektrode und der im ersten Speicher gespeicherten ersten Eichkurve,
(f3) Berechnen des Ausgangsniveaus in der zweiten Enzymelektrode (6) der zu messenden ersten Komponente auf der Basis der berechneten Konzentration der zu messenden ersten Komponente und der im zweiten Speicher (12) gespeicherten Eichkurve,
(f4) Berechnen des Ausgangsniveaus in der zweiten Enzymelektrode (6) der zu messenden zweiten Komponente durch Substrahieren des berechneten Ausgangsniveaus in der zweiten Enzymelektrode (6) der zu messenden ersten Komponente vom Ausgangsniveau in der zweiten Enzymelektrode (6) der Standardlösung, und
(g) eine Verarbeitungseinrichtung (5) zum Berechnen der Konzentration der zu messenden ersten Komponente und der zu messenden zweiten Komponente der Probe durch Verwenden der ersten, zweiten und dritten Eichkurven.

3. Zweikomponentenvorrichtung nach Anspruch 1 oder 2, wobei die zweite Enzymelektrode (6) auf der stromab gelegenen Seite der ersten Enzymelektrode (4) angeordnet ist und eine Mischspule (5) zwischen der ersten Enzymelektrode (4) und der zweiten Enzymelektrode (6) angeordnet ist zum Verteilen und Verdünnen der zu messenden ersten und zweiten Komponenten in den Vorwärts- und Rückwrätsrichtungen der Strömung.

4. Zweikomponenetenmeßverfahren umfassend
(a) einen ersten Schritt zum Bereiten
(a1) einer ersten Enzymelektrode, die nur auf eine zu messende erste Komponente anspricht, und
(a2) einer zweiten Enzymelektrode, die sowohl auf die zu messende erste Komponente als auch auf die zu messende zweite Komponente anspricht,
(b) einen zweiten Schritt zum Berechnen einer ersten Eichkurve für die Beziehung zwischen der Konzentration der zu messenden ersten Komponente und dem Ausgangsniveau der ersten Enzymelektrode (4),
(c) einen dritten Schritt zum Berechnen einer zweiten Eichkurve für die Beziehung zwischen der Konzentration der zu messenden ersten Komponente und dem Ausgangsniveau der zweiten Enzymelektrode (6),
(d) einen vierten Schritt zum Bereiten einer Standardlösung, die sowohl die zu messende erste Komponente als auch die zu messende zweite Komponente enthält, mit einer bekannten Konzentration der zu messenden zweiten Komponente,
(e) einen fünften Schritt zum Bestimmen der Konzentration der in der ersten Standardlösung enthaltenen, zu messenden ersten Komponente durch Verwenden des Ausgangsniveaus der ersten Enzymelektrode (4) der Standardlösung und der ersten Eichkurve,
(f) einen sechsten Schritt zum Bestimmen des Ausgangsniveaus in der zweiten Enzymelektrode (6) der in der Standdardlösung enthaltenen, zu messenden ersten Komponente durch Verwenden der Konzentration der zu messenden ersten Komponente und der zweiten Eichkurve,
(g) einen siebten Schritt zum Bestimmen des Ausgangsniveaus in der zweiten Enzymelektrode (6) der zu messenden zweiten Komponente durch Substrahieren des Ausgangsniveaus in der zweiten Enzymelektrode (6) der zu messenden ersten Komponente vom Ausgangsniveau der zweiten Enzymelektrode,
(h) einen achten Schritt zum Berechnen einer dritten Eichkurve für die Beziehung zwischen der Konzentration der zu messenden zweiten Komponente und dem Ausgangsniveau der zweiten Enzymelektrode (6) durch Variieren der Konzentration der zu messenden zweiten Komponente in der Standardlösung und Wiederholen des Vorgangs aufeinanderfolgend vom vierten bis zum siebten Schritt und
(i) einen neunten Schritt zum Bestimmen der Konzentrationen der zu messenden ersten und zweiten Komponenten auf der Basis der ersten, zweiten und dritten Eichkurven.

## Revendications

1. Appareil destiné à mesurer deux composants, comprenant :
une première électrode enzymatique (4) qui ne réagit qu'à un premier composant à mesurer, et une deuxième électrode enzymatique (6) qui réagit à la fois au premier composant à mesurer et au deuxième composant à mesurer, les deux électrodes enzymatiques (4, 6) possédant une enzyme commune dans la réaction enzymatique servant à former une substance détectable au niveau des électrodes, identique dans la substance devant être finalement détectée par les électrodes, et comprenant en outre un moyen qui permet de calculer une courbe d'étalonnage du deuxième composant à mesurer, comprenant les opérations suivantes :
(i) calculer la partie contributrice du premier composant à mesurer, qui est une impureté de la solution étalon contenant une quantité connue du deuxième composant, dans la deuxième électrode enzymatique (6) sur la base de la relation suivante :
(A) le niveau de sortie de la première électrode enzymatique (4) provoqué par une solution étalon du deuxième composant à mesurer contenant le premier composant au titre d'impureté, et
(B) la relation des niveaux de sortie respectifs de la première électrode enzymatique (4) et de la deuxième électrode enzymatique (6), provoqués par une solution étalon du premier composant à mesurer ; et
(ii) soustraire ladite partie contributrice du niveau de sortie de la solution étalon contenant une quantité connue du deuxième composant dans la deuxième électrode enzymatique (6).

2. Appareil destiné à mesurer deux composants, comprenant :
a. une première électrode enzymatique (4) qui ne réagit qu'à un premier composant à mesurer;
b. une deuxième électrode enzymatique (6) qui répond à la fois au premier composant à mesurer et au deuxième composant à mesurer;
c. une première mémoire (11) servant à mémoriser une première courbe d'étalonnage permettant d'exprimer la relation entre la concentration en le premier composant à mesurer et le niveau de sortie de la première électrode enzymatique (4);
d. une deuxième mémoire (12) servant à mémoriser une deuxième courbe d'étalonnage permettant d'exprimer la relation entre la concentration en le premier composant à mesurer et le niveau de sortie de la deuxième électrode enzymatique (6);
e. une troisième mémoire (13) servant à mémoriser une troisième courbe d'étalonnage permettant d'exprimer la relation entre la concentration en le deuxième composant à mesurer et le niveau de sortie de la deuxième électrode enzymatique (6) tel que calculé par f;
f. un moyen de traitement (14) servant à calculer la troisième courbe d'étalonnage, ledit moyen de traitement (14) calculant la troisième courbe d'étalonnage sur la base des niveaux de sortie dans la deuxième électrode enzymatique (6) du deuxième composant obtenus par modification de la concentration en le deuxième composant à mesurer dans une solution étalon, et répétant le procédé suivant la séquence f1 à f4;
f1. détecter les niveaux de sortie pouvant être attribués à la solution étalon dans la première électrode enzymatique (4) et la deuxième électrode enzymatique (6), ladite solution étalon contenant le premier composant à mesurer et le deuxième composant à mesurer sous une concentration connue du deuxième composant ;
f2. calculer la concentration en le premier composant à mesurer sur la base du niveau de sortie détecté dans la première électrode enzymatique (4) et de la première courbe d'étalonnage mémorisée dans la première mémoire (11);
f3. calculer le niveau de sortie dans la deuxième électrode enzymatique (6) du premier composant à mesurer sur la base de la concentration calculée en le premier composant à mesurer et de la deuxième courbe d'étalonnage mémorisée dans la deuxième mémoire (12) ;
f4. calculer le niveau de sortie dans la deuxième électrode enzymatique (6) du deuxième composant à mesurer en soustrayant le niveau de sortie calculé dans la deuxième électrode enzymatique (6) du premier composant à mesurer vis-à-vis du niveau de sortie dans la deuxième électrode enzymatique (6) de la solution étalon ; et
g. un moyen de traitement (15) servant à calculer les concentrations en le premier composant à mesurer et en le deuxième composant à mesurer de l'échantillon à l'aide des première, deuxième et troisième courbes d'étalonnage.

3. Appareil destiné à mesurer deux composants selon la revendication 1 ou 2, où la deuxième électrode enzymatique (6) est disposée du côté amont de la première électrode enzymatique (4), et
un serpentin de mélange (5) est disposé entre la première électrode enzymatique (4) et la deuxième électrode enzymatique (6) afin de faire diffuser et diluer les premier et deuxième composants à mesurer dans les directions avant et arrière de l'écoulement.

4. Procédé permettant de mesurer deux composants, comprenant :
a. une première opération de préparation de :
a1. une première électrode enzymatique (4) ne réagissant qu'à un premier composant à mesurer, et
a2. une deuxième électrode enzymatique (6) répondant à la fois au premier composant à mesurer et au deuxième composant à mesurer,
b. une deuxième opération de calcul d'une première courbe d'étalonnage permettant d'exprimer la relation entre la concentration en le premier composant à mesurer et le niveau de sortie de la première électrode enzymatique (4),
c. une troisième opération de calcul d'une deuxième courbe d'étalonnage permettant d'exprimer la relation entre la concentration en le premier composant a mesurer et le niveau de sortie de la deuxième électrode enzymatique (6),
d. une quatrième opération de préparation d'une solution étalon contenant à la fois le premier composant à mesurer et le deuxième composant à mesurer, la concentration du deuxième composant à mesurer étant connue,
e. une cinquième opération de détermination de la concentration en le premier composant à mesurer contenu dans la solution étalon, à l'aide du niveau de sortie de la première électrode enzymatique (4) pour la solution étalon et de la première courbe d'étalonnage,
f. une sixième opération de détermination du niveau de sortie dans la deuxième électrode enzymatique (6) du premier composant à mesurer contenu dans la solution étalon, à l'aide de la concentration en le premier composant à mesurer et de la deuxième courbe d'étalonnage,
g. une septième opération de détermination du niveau de sortie dans la deuxième électrode enzymatique (6) du deuxième composant à mesurer, que l'on obtient en soustrayant le niveau de sortie dans la deuxième électrode enzymatique (6) du premier composant à mesurer vis-à-vis du niveau de sortie de la deuxième électrode enzymatique (6),
h. une huitième opération de calcul d'une troisième courbe d'étalonnage permettant d'exprimer la relation entre la concentration en le deuxième composant à mesurer et le niveau de sortie de la deuxième électrode enzymatique (6), que l'on obtient en faisant varier la concentration en le deuxième composant à mesurer dans la solution étalon et en répétant le processus dans la séquence des opérations allant de la quatrième à la septième, et
i. une neuvième opération de détermination des concentrations en les premier et deuxième composants à mesurer sur la base des première, deuxième et troisième courbes d'étalonnage.
